Europäisches Patentamt

European Patent Office

Office européen des brevets

(19)

(11)     EP 1 246 577 B1

(12)     **EUROPÄISCHE PATENTSCHRIFT**

(45) Veröffentlichungstag und Bekanntmachung des Hinweises auf die Patenterteilung:
**01.09.2004   Patentblatt 2004/36**

(21) Anmeldenummer: **00900061.3**

(22) Anmeldetag: **13.01.2000**

(51) Int Cl.⁷: **A61B 17/70**

(86) Internationale Anmeldenummer:
**PCT/CH2000/000018**

(87) Internationale Veröffentlichungsnummer:
**WO 2001/050968 (19.07.2001 Gazette 2001/29)**

(54) **VORRICHTUNG ZUM LÖSBAREN FESTKLEMMEN EINES LÄNGSTRÄGERS INNERHALB EINES CHIRURGISCHEN IMPLANTATES**

DEVICE FOR DETACHABLY CLAMPING A LONGITUDINAL CARRIER IN A SURGICAL IMPLANT

DISPOSITIF POUR SERRER DE MANIERE LIBERABLE UN SUPPORT OBLONG DANS UN IMPLANT CHIRURGICAL

(84) Benannte Vertragsstaaten:
**AT BE CH CY DE DK ES FI FR GB GR IE IT LI LU MC NL PT SE**

(43) Veröffentlichungstag der Anmeldung:
**09.10.2002   Patentblatt 2002/41**

(73) Patentinhaber: **SYNTHES AG Chur**
**7002 Chur (CH)**

(72) Erfinder:
• **SCHÄR, Manuel**
**CH-4132 Muttenz (CH)**

• **GERBER, David**
**CH-9320 Arbon (CH)**
• **SCHLÄPFER, Fridolin**
**CH-8750 Glarus (CH)**

(74) Vertreter: **Lusuardi, Werther Giovanni, Dr.**
**Dr. Lusuardi AG,**
**Kreuzbühlstrasse 8**
**8008 Zürich (CH)**

(56) Entgegenhaltungen:
**EP-A- 0 836 836          WO-A-99/09901**
**FR-A- 2 736 535**

**Beschreibung**

Die Erfindung bezieht sich auf eine Vorrichtung zum lösbaren Festklemmen und Verbinden eines Längsträgers innerhalb eines chirurgischen Implantates im menschlichen oder tierischen Körper gemäss dem Oberbegriff des Patentanspruchs 1.

Chirurgische Implantate, wie sie zur Knochenfixation oder im speziellen zur Wirbelsäulenfixation angewendet werden, enthalten vielfach einen oder mehrere Längsträger, woran dann die Knochenfixationselemente oder speziell bei der Wirbelsäulenfixation die Pedikelschrauben befestigt und damit in einer longitudinalen Richtung gegeneinander fixiert werden. Zur Versteifung eines gesamten, mehrere Längsträger enthaltenden Implantates werden die Längsträger mittels Querverbindern, welche quer zu den Längsträgern zwischen diesen angebracht werden, miteinander verbunden.

Aus dem Stand der Technik sind bereits einige Vorrichtungen zur Verbindung von Längsträgern innerhalb von Wirbelsäulenimplantaten bekannt. Diese besitzen den Vorteil, dass die implantierten Längsträger durch die Querverbindung mittels dieser Vorrichtungen erheblich versteift werden. Auch ist es dank der bereits bekannten Vorrichtungen möglich, die Querverbindungen unter Winkeln, die in einem bestimmten Mass vom rechten Winkel abweichen, zu implantieren.

Eine solche Verbindungsvorrichtung von Längsträgern ist beispielsweise aus der EP-A 0 836 836 RICHELSOPH bekannt. Die dort offenbarte Vorrichtung zur Verbindung von Längsträgern innerhalb von Wrrbelsäulenimplantaten umfasst einen teleskopierbaren Querverbindungskörper mit zwei Bohrungen zur Aufnahme je eines Einsatzes, wobei die beiden Bohrungen an den entgegengesetzten Enden des Querverbindungskörpers angebracht sind. Ebenfalls an den Enden ist je eine Ausnehmung angebracht, deren Zentratachsen quer zu den Längsachsen der Bohrungen verlaufen und diese schneiden. Diese Ausnehmung ermöglichen ein teilweises Überstülpen des Querverbindungskörpers über die quer zu dessen Zentralachse verlaufenden Längsträger. Die Bohrungen sind auf einem Abschnitt ihrer Länge mit einem Konus versehen, während jeder in eine Bohrung einzuführende Einsatz auf einer Teillänge seiner äusseren Mantelfläche einen zum Konus in der Bohrung entsprechenden Aussenkonus aufweist. Die Einsätze sind mittels quer zu den Längsachsen der Bohrungen angebrachter Öffnungen jochartig mit federbaren Schenkeln ausgebildet. Die Öffnungen in den Einsätzen dienen zur Aufnahme der Längsträger und sind so gestaltet, dass die federbaren Schenkel den Längsträger mehr als halbkreisförmig umschliessen, jedoch durch die federnden Schenkel ein Einführen beziehungsweise Entfernen des Längsträgers ermöglicht wird. Blockiert werden Einsatz und Längsträger gleichzeitig durch Anziehen eines Feststellmittels, weiches über oder in ein Gewinde am an das Joch anschliessenden Teil des Einsatzes geschraubt wird. Beim Anziehen dieses Feststellmittels wird der Einsatz in die Bohrung im Querverbindungskörper hineingezogen, wobei die Schenkel des Einsatzes durch die Konusverbindung zusammengepresst werden und somit der Längsträger in der Durchgangsöffnung innerhalb der Schenkel und der Einsatz im Querverbindungskörper blockiert werden.

Eine weitere solche Verbindungsvorrichtung ist aus der EP-A 0 778 007 PFAIFER bekannt. Diese bekannte Vorrichtung umfasst einen Zentralkörper, einen über diesen schiebbaren Ring und eine auf ein am oberen Ende des Zentralkörpers angebrachtes Aussengewinde schraubbare Mutter. Am unteren Ende ist der Zentralkörper mit einem Kanal versehen, dessen Zentralachse quer zur Längsachse des Zentralkörpers verläuft, so dass am Zentralkörper zwei einander gegenüberliegende federbare Klauen zur Aufnahme des Längsträgers gebildet werden. Die zylindrische Aussenform des Zentralkörpers weist auf einem Teil ihrer Länge einen grösseren Durchmesser auf, wodurch im Bereich der federbaren Klauen eine auf der Mantelfläche umlaufende Schulter gebildet wird. Der Bohrung des Ringes ist auf ihrer gegen den Kanal gerichteten Seite mit einem gegen das Ringende weiter werdenden Konus versehen, so dass beim Überschieben des Ringes über den Zentralkörper, der Konus an der Schulter ansteht und bei Aufbringen einer axialen Kraft mittels Anziehen der Mutter der Konus teilweise über die Schulter geschoben wird. Dadurch werden die federbaren Klauen gegen innen zusammengepresst und ein darin eingespannter Längsträger im Kanal fixiert. Quer zur Längsachse des Zentralkörpers und quer zur Zentralachse des Kanals ist im Zentralkörper eine Durchgangsöffnung angebracht, welche die Aufnahme eines Querstabes ermöglicht. Dieser Querstab wird in einen entsprechenden Kanal im Ring aufgenommen und gegenüber der Vorrichtung durch Einklemmen zwischen Ring und Mutter fixiert.

Nachteilig bei der EP-A 0 778 007 PFAIFER ist, dass der Längsträger nur innerhalb eines kleinen, den rechten Winkel einschliessenden Winkelbereichs gegenüber dem Querverbinder angeordnet werden kann. Die in der EP-A 0 836 836 RICHELSOPH offenbarte Vorrichtung lässt einen grösseren Winkelbereich zwischen Längsträger und Querverbinder zu, bietet jedoch gegen nachträgliches Verdrehen durch auf das Implantat wirkende Kräfte nur eine geringe Sicherheit.

Eine weitere Vorrichtung zur Verbindung von Längsträgern ist aus der FR 2 736 535 MARTIN bekannt. Diese bekannte Vorrichtung umfasst eine Klemmkonusverbindung zur Fixierung eines Längsträgers im Verbindungsteil mit einem kurzen Innenkonus, welcher über den einen Aussenkonus aufweisenden, den Längsträger in seiner Durchgangsöffnung festklemmenden Klemmkörper geschoben wird. Nachteilig an dieser bekannten Vorrichtung ist die geringe Stabilität der Vorrichtung gegen Verdrehung der Längsträger um die Zentralachse der Klemmkörper.

Eine andere Vorrichtung zur Verbindung eines Längsträgers mit einem weiteren Implantat ist aus der WO 99/09901 TAGWERKER bekannt Auch diese bekannte Vorrichtung umfasst eine Klemmkonusverbindung zur Fixierung eines Längsträgers im Verbindungsteil. Durch eine Ausgestaltung des Verbindungsteiles für grosse Drehwinkel des Längsträgers um die Zentralachse des Klemmkörpers wird die Stabilität der Klemmkonusverbindung verringert.

Hier will die Erfindung Abhilfe schaffen. Der Erfindung liegt die Aufgabe zugrunde, eine Vorrichtung zur Verbindung von Stäben und speziell auch Längsträgern von Wirbelsäulenimplantaten oder auch zur Verbindung eines Stabes mit einem weiteren Implantat, beispielsweise mit einem Knochenverankerungselement oder einer Pedikelschraube mit den folgenden Eigenschaften zu schaffen:

- einfache Handhabung;
- Verbindung von Längsträgern, die nicht parallel implantiert sind;
- Verhindern eines Verrutschens der Stabverbindung nach der Implantation; und
- Ermöglichen eines sehr steifen gesamten Implantates, auch dadurch, dass sich die Längsträger nach der Befestigung der Stabverbindungen nicht verdrehen können.

Die Erfindung löst die gestellte Aufgabe mit einer Vorrichtung zum lösbaren Festklemmen und Verbinden eines Längsträgers innerhalb eines chirurgischen Implantates im menschlichen oder tierischen Körper, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung aufgrund der gezielten Krafteinwirkung auf den Längsträger ein Verrutschen der Stabverbindung nach der Implantation verhindert wird, eine Verbindung von Längsträgem, die nicht parallel implantiert sind, auch bei Auftreten grosser durch den oder die Längsträger ausgeübter Drehmomente auf die Vorrichtung möglich ist und das gesamte Implantat eine hohe Steifigkeit aufweist.

Die erfindungsgemässe Vorrichtung umfasst mindestens einen jochartigen Verbindungskörper mit in Richtung einer Zentralachse verlaufenden Seitenwänden, wobei der zwischen diesen Seitenwänden liegende Freiraum zur Durchführung des Längsträgers quer zur Zentralachse dient, und einer koaxial zur Zentralachse verlaufenden Durchgangsbohrung. Quer zur Zentralachse ist am Verbindungskörper ein Kupplungsmittel, beispielsweise ein Querstab angeordnet, welcher zur Verbindung mit einem weiteren Teil eines Implantates, beispielsweise einer weiteren erfindungsgemässen Vorrichtung, einem Knochenverankerungselement oder einer Pedikelschraube dient. Im weiteren umfasst die Vorrichtung einen jochartigen Klemmkörper, welcher in der Durchgangsbohrung gleitbar angeordnet ist und dessen Seitenwände im Bereich des Joches in Richtung der Zentralachse verlaufen und quer zur Zentralachse federbar sind. Die zwischen diesen Seitenwänden liegende, zum Freiraum korrespondierende Durchgangsöffnung dient zur Aufnahme des Längsträgers. Zur Blokkierung des Längsträgers und des Klemmkörpers innerhalb des Verbindungskörpers umfasst die Vorrichtung ein Fixationsmittel. Die Seitenwände des Klemmkörpers sind an ihren freien Enden mit gegen das Innere der Durchgangsöffnung gerichteten Erhebungen versehen. Diese Erhebungen verengen den Querschnitt der Durchgangsöffnung so, dass der Längsträger in der Durchgangsöffnung in Richtung der Zentralachse festgehalten wird und beim Anziehen des Fixationsmittels die Seitenwände durch die von mindestens einem der relativ zum Längsträger bewegten Teile der Vorrichtung ausgeübten Druckkraft des Längsträgers auf die Erhebungen gegen die Wand der Durchgangsbohrung gepresst werden. Dadurch werden beim Anziehen des Fixationsmittels der Längsträger und der Klemmkörper gleichzeitig im Verbindungskörper fixiert.

In einer bevorzugten Ausführungsform der erfindungsgemässen Vorrichtung sind aussen am Klemmkörper und in der Durchgangsbohrung korrespondierende Verzahnungen angebracht, deren Zähne parallel zur Zentralachse verlaufen. Diese Verzahnungen gestatten, dass einerseits der Klemmkörper im Verbindungskörper unter verschiedenen Drehwinkeln um die Zentralachse montierbar ist und andererseits der Klemmkörper im montierten und implantierten Zustand auch bei Auftreten grösserer Drehmomente durch den im Klemmkörper befestigten Längsträger sich gegenüber dem Verbindungskörper nicht verdrehen kann.

Vorteilhafterweise ist das Fixationsmittel so gestaltet, dass vom fixationsmittelseitigen Ende des Verbindungskörpers eine Zugkraft auf den Klemmkörper ausübbar ist. Dazu ist beispielsweise der Klemmkörper an seinem fixationsmittelseitigen Ende mit einem koaxial zur Zentralachse verlaufenden Innengewinde versehen und das Fixationsmittel als Feststellschraube ausgeführt, welche ein zum Innengewinde korrespondierendes Aussengewinde aufweist und deren Schraubenkopf am fixationsmittelseitigen Ende des Verbindungskörpers gegen eine Bewegung in Richtung der Zentralachse gesichert ist. Die Sicherung des Schraubenkopfes ist durch Lagerung des Schraubenkopfes oder eines flanschförmigen Teiles davon in einer Hinterdrehung in der Durchgangsbohrung des Verbindungskörpers möglich. Dadurch ist die Feststellschraube gegen Bewegungen in axialer Richtung gesichert und weiterhin frei drehbar.

In einer weiteren bevorzugten Ausführungsform der erfindungsgemässen Vorrichtung ist der Verbindungskörper so ausgebildet, däss die Feststellschraube in Richtung der Zentralachse um eine Länge X vom fixationsmittelseitigen Ende her in den Verbindungskörper eindringt, der Verbindungskörper in Richtung der Zentralachse zwischen seinem fixationsmittelseitigen Ende und seinem klemmkörperseitigen Ende eine Höhe H aufweist, der Freiraum in Richtung der Zentralachse zwischen dem klemmkörperseitigen Ende und seiner oberen, in Richtung des fixationsmittelseitigen Endes liegenden Begrenzung eine Tiefe T aufweist und die Bedingung

$$X > H - T$$

erfüllt ist. Durch Erfüllung dieser Bedingung wird gewährleistet, dass nur das klemmkörperseitige Ende der Feststellschraube von oben auf den Längsträger drückt und diesen gegen die Erhebungen an den Seitenwänden des Klemmkörpers presst.

In wieder einer weiteren bevorzugten Ausführungsform der erfindungsgemässen Vorrichtung ist der Verbindungskörper so ausgebildet, dass die Feststellschraube in Richtung der Zentralachse nur um eine Länge X vom fixationsmittelseitigen Ende her in den Verbindungskörper eindringen kann, der Verbindungskörper in Richtung der Zentralachse zwischen seinem fixationsmittelseitigen Ende und seinem klemmkörperseitigen Ende eine Höhe H aufweist, der Freiraum in Richtung der Zentralachse zwischen dem klemmkörperseitigen Ende und seiner oberen, in Richtung des fixationsmittelseitigen Endes liegenden Begrenzung eine Tiefe T aufweist und die Bedingung

$$X < H - T$$

erfüllt ist. Durch Erfüllung dieser Bedingung wird gewährleistet, dass nur die obere Begrenzung des Freiraumes von oben auf den Längsträger drückt und diesen gegen die Erhebungen an den Seitenwänden des Klemmkörpers presst.

Anstelle der Ausgestaltung des Fixationsmittels als Feststellschraube ist auch die Ausgestaltung des Fixationsmittels als Mutter möglich, welche über einen entsprechend am Klemmkörper angebrachten Gewindezapfen geschraubt wird.

Die Durchgangsöffnung weist vorzugsweise einen kreiszylindrischen Kern mit einem Durchmesser D auf, während die die Erhebungen gegen die Durchgangsöffnung begrenzenden Seitenflächen einen Abstand A aufweisen. Das Verhältnis A/D beträgt zwischen 85 % und 98 %, vorzugsweise zwischen 92 % und 98 %. Diese Ausgestaltung der Durchgangsöffnung ermöglicht durch die federbaren Seitenwände des Klemmkörpers ein Einschnappen des Längsträgers und eine stabile Fixierung des Längsträgers in der Durchgangsöffnung beim Anziehen der Feststellschraube.

Durch die Ausgestaltung keilförmiger Erhebungen lässt sich die Klemmkraft des Klemmkörpers innerhalb der Durchgangsbohrung im Verbindungskörper optimieren. Die Erhebungen sind derart beschaffen, dass sich jede Erhebung in Richtung der Zentralachse und gegen das Innere der Durchgangsöffnung verjüngt. Die Keilform der Erhebungen lässt sich auch durch einen beispielsweise einen kreissegmentartigen Querschnitt aufweisenden Kern der Durchgangsöffnung mit einem symmetrisch zur Zentralachse gemessenen Zentriwinkel von mehr als 180° erreichen. Vorzugsweise weist die Durchgangsöffnung im Querschnitt einen Zentriwinkel von zwischen 200° und 250°, insbesondere von zwischen 210° und 240°, auf.

In einer weiteren Ausführungsform umfasst die erfindungsgemässe Vorrichtung zwei Verbindungskörper mit je einem Klemmkörper und je einer Feststellschraube, wobei die beiden Verbindungskörper durch Querstäbe, welche ineinander teleskopierbar sind, miteinander verbunden sind. Diese Ausführungsform der erfindungsgemässen Vorrichtung eignet sich als Querverbinder zwischen zwei Längsträgern beispielsweise innerhalb eines Wirbelsäulenimplantates.

Anstelle der teleskopierbaren Querstäbe sind auch Muffen, welche als Kupplungsmittel an den Verbindungskörpern angebracht sind, zur verschiebbaren Aufnahme eines Querstabes denkbar.

Damit der Längsträger vom Eintritt her in Richtung der Zentralachse einfacher in die Durchgangsöffnung einführen lässt, können die freien Enden des Klemmkörpers auch mit Backen versehen sein, welche so beschaffen sind, dass sich der Querschnitt der Durchgangsöffnung vom Eintritt her gegen die Erhebungen verengt.

Weitere Anwendungsmöglichkeiten bestehen darin, dass ein Verbindungskörper mittels des Querstabes beispielsweise mit einem Knochenverankerungselement oder einer Pedikelschraube verbunden ist.

Die Erfindung und Weiterbildungen der Erfindung werden im folgenden anhand der teilweise schematischen Darstellungen mehrerer Ausführungsbeispiele noch näher erläutert.

Es zeigen:

Fig. 1 eine perspektivische Darstellung einer Ausführungsform der erfindungsgemässen Vorrichtung;

Fig. 2 einen Schnitt durch eine Variante der in Fig. 1 dargestellten Ausführungsform der erfindungsgemässen Vorrichtung;

Fig. 3 einen Schnitt durch eine weitere Variante der in Fig. 1 dargestellten Ausführungsform der erfindungsgemässen Vorrichtung;

Fig. 4 eine perspektivische Darstellung einer weiteren Ausführungsform der erfindungsgemässen Vorrichtung;

Fig. 5 einen Schnitt durch die in Fig. 4 dargestellte Ausführungsform der erfindungsgemässen Vorrichtung;

Fig. 6 eine perspektivische Darstellung einer weiteren Ausführungsform der erfindungsgemässen Vorrichtung;

Fig. 7 einen Schnitt durch die in Fig. 6 dargestellte Ausführungsform der erfindungsgemässen Vorrichtung; und

Fig. 8 einen Schnitt durch eine weitere Ausführungsform der erfindungsgemässen Vorrichtung.

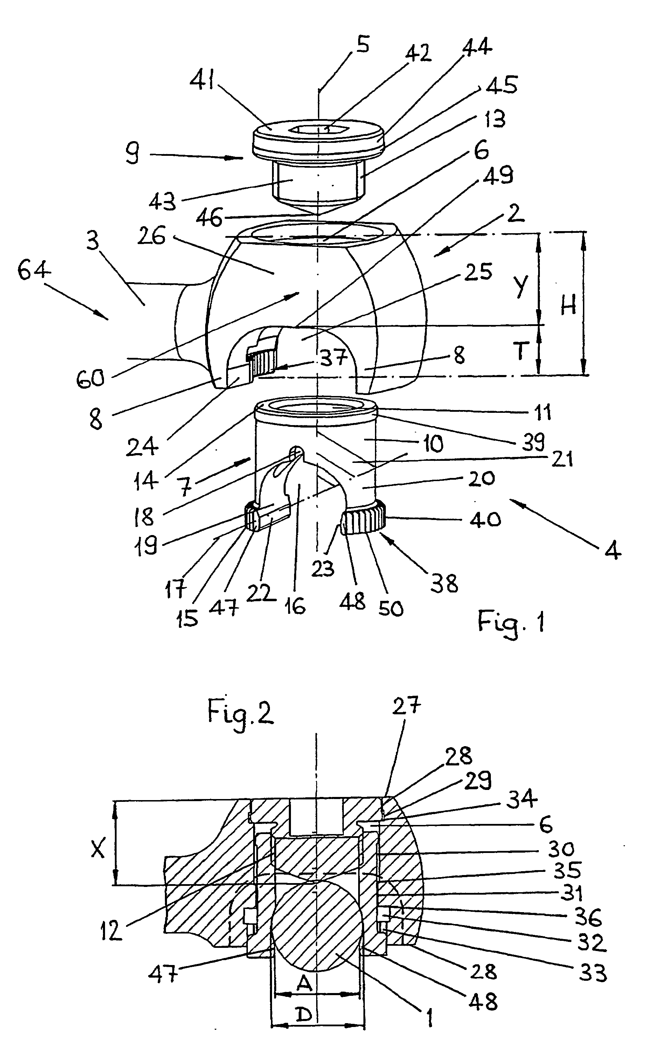

In den Fig. 1 und 2 ist die erfindungsgemässe Vorrichtung in einer Ausführungsform als Verbindungselement 4 zwischen einem Längsträger 1 und einem Querstab 3 dargestellt. Die Vorrichtung umfasst in dieser Ausführungsform einen jochartigen, zu einer Zentralachse 5 symmetrischen Verbindungskörper 2 mit einer zur Zentralachse 5 koaxialen Durchgangsbohrung 6, einen hohlzylindrischen Klemmkörper 7, welcher in der Durchgangsbohrung 6 parallel zur Zentralachse 5 verschiebbar ist, und ein Fixationsmittel 9 zur Fixierung des Längsträgers 1 innerhalb der Vorrichtung.

Der jochartige Verbindungskörper 2 ist innerhalb der Vorrichtung so angeordnet, dass die beiden Seitenwände 8 in Richtung der Zentralachse 5 verlaufen. Der innerhalb der Seitenwände 8 liegende Freiraum 25 weist gegen die Durchgangsbohrung 6 hin konvergierende Seitenflächen 24 auf, womit erreicht wird, dass der durch den Freiraum 25 durchgehende Längsträger 1 innerhalb eines Winkelbereichs von zwischen 75° und 105° zur Stirnfläche 26 des Verbindungskörpers 2 in diesem befestigbar ist. Die Durchgangsbohrung 6 ist mit im Durchmesser verschiedenen Abschnitten 28;29;30; 31 ;32;33 derart ausgeführt, dass am fixationsmittelseitigen Ende 27 ein zylindrischer Abschnitt 28 mit einer Hinterdrehung 29 in einen einen geringeren Durchmesser aufweisenden Abschnitt 30 mündet, wodurch eine erste Schulter 34 entsteht, anschliessend der Abschnitt 30 wiederum in einen einen geringeren Durchmesser aufweisenden Abschnitt 31 mündet, wodurch eine zweite Schulter 35 entsteht, der Abschnitt 31 in eine zweite Hinterdrehung 32 mündet, und daran anschliessend bis zum klemmkörperseitigen Ende 28 ein mit einer parallel zur Zentralachse 5 verlaufenden Verzahnung 37 versehener Abschnitt 33 folgt. Der Aussendurchmesser des verzahnten Abschnittes 33 ist grösser als der Durchmesser des Abschnittes 31, so dass eine dritte Schulter 36 entsteht. Die beiden Schultern 34 und 35 dienen als Anschläge in Richtung des klemmkörperseitigen Endes 28, während die als Fixationsmittel 9 dienende Feststellschraube 43 auch als Anschlag in Richtung des fixationsmittelseitigen Endes 27 dient. Aussen am Verbindungskörper 2 ist senkrecht zur Zentralachse 5 ein Querstab 3 angebracht, welcher eine Verbindung zu anderen Teilen des gesamten Implantates ermöglicht.

Der ebenfalls jochartige Klemmkörper 7 besteht aus einem konzentrisch zur Zentralachse 5 verlaufenden Hohlzylinder 10 mit freien, längsträgerseitigen Enden 15;50, einem oberen, fixationsmittelseitigen Ende 14 und einer Durchgangsöffnung 16 mit einer rechtwinklig zur Zentralachse 5 vorlaufenden Längsachse 17. Die Durchgangsöffnung 16 ist gegen das untere Ende 15 mittels keilartiger, sich gegen das obere Ende 14 verjüngenden Erhebungen 47 und 48 verengt und offen, so dass der Klemmkörper 7 eine gegen die freien Enden 15;50 offene, U-artige Gestalt mit gegen die Durchgangsöffnung 16 hin klammerförmig ausgebildeten Seitenwänden 19;20 erhält. Die Durchgangsöffnung 16 ist symmetrisch zu der Ebene 21, welche die Zentralachse 5 und die Längsachse 17 enthält, und umfasst einen zylindrischen Kern mit dem Durchmesser D zur Aufnahme des Längsträgers 1. Gegen das obere Ende 14 hin mündet die Durchgangsöffnung 16 in einen zur Ebene 21 parallelen, gegen das obere Ende 14 abgeschlossenen Schlitz 18 und gegen das unteren Ende 15 hin in einen Teil mit zur Ebene 21 parallelen, die Erhebungen 47;48 begrenzenden Seitenflächen 22;23. Der rechtwinklig zur Ebene 21 betrachtete Abstand A zwischen diesen beiden Seitenflächen 22;23 ist kleiner als der Durchmesser D ist und beträgt in der hier beschriebenen Ausführungsform 95 % des Durchmessers D. Der Klemmkörper 7 weist einen an die längsträgerseitigen, unteren Enden 15;50 anschliessenden Abschnitt 40 mit einer Verzahnung 38 auf, welche zur Verzahnung 37 im Verbindungskörper 2 korrespondiert. An das fixationsmittelseitige, obere Ende 14 anschliessend ist ein Abschnitt 39, welcher einen grösseren Durchmesser als der Hohlzylinder 10 aufweist, wobei der Durchmesser dieses Abschnittes 39 mit dem Durchmesser des Abschnittes 30 im Verbindungskörper 2 korrespondiert, so dass ein Herausrutschen des Klemmkörpers 7 in Richtung des unteren Endes 28 des Verbindungskörpers 2 im montierten Zustand durch die zweite Schulter 35 verhindert wird. Zur Montage des Klemmkörpers 7 wird der Abschnitt 39 des Klemmkörpers 7 durch den einen kleineren Durchmesser aufweisenden Abschnitt 31 im Verbindungskörper 2 durchgepresst, so dass der Abschnitt 39 elastisch deformiert wird und im Abschnitt 30 wieder ausfedert.

Ein Herausrutschen des Klemmkörpers 7 in Richtung des oberen Endes 27 des Verbindungskörpers 2 wird durch die Feststellschraube 43 verhindert. Die Verzahnungen 37;38 gestatten, dass einerseits der Klemmkörper 7 im Verbindungskörper 2 unter verschiedenen Drehwinkeln um die Zentralachse 5 montierbar ist und andererseits der Klemmkörper 7 im montierten Zustand auch bei Auftreten grösserer Drehmomente durch den im Klemmkörper 7 befestigten Längsträger 1 sich gegenüber dem Verbindungskörper 2 nicht verdrehen kann. Zudem ist die Bohrung 11 im Klemmkörper 7 an ihrem an das obere Ende 14 anschliessenden Teil mit einem Innengewinde 12 versehen.

Das Fixationsmittel 9 ist als Feststellschraube 43 mit einem zum Innengewinde 12 korrespondierenden Aussengewinde 13, einem Schraubenkopf 41 und einem Innensechskant 42 ausgebildet. Der Schraubenkopf 41 ist zylindrisch gestaltet und weist zwei Abschnitte 44;45 auf, wobei der gewindeseitige Abschnitt 45 einen grösseren Durchmesser aufweist, so dass dieser Abschnitt 45 in die Hinterdrehung 29 einrastet und somit die Feststellschraube 43 in Richtung der Zentralachse 5 fixiert ist, jedoch um die Zentralachse 5 frei drehbar ist. Zur Montage der Feststellschraube 43 wird der Abschnitt 45 der Feststellschraube 43 durch den einen kleineren Durchmesser aufweisenden Abschnitt 28 im Verbindungskörper 2 durchgepresst, so dass der Abschnitt 45 elastisch deformiert wird und im Abschnitt 29 wieder ausfedert.

Die klammerartigen und federbaren Seitenwände 19;20 des Klemmkörpers 7 gestatten ein Einschnappen des Längsträgers 1 vom unteren Ende 15 her. Ist der Längsträger 1 einmal in die Durchgangsöffnung 16 eingelegt, wird der Klemmkörper 7 durch Anziehen der Feststellschraube 43 in die Durchgangsbohrung 6 des Verbindungskörpers 2 so weit eingezogen, bis das klemmkörperseitige Ende 46 der Feststellschraube 43 auf den Längsträger 1 drückt. Bei weiterem Anziehen der Feststellschraube 43 wird der Längsträger 1 mit einer Kraft F gegen die Erhebungen 47;48 gepresst. Der kreisförmige Querschnitt des Längsträgers 1 und die Keilform der Erhebungen 47;48 bewirken, dass die Kraft F auch eine senkrecht zur Zentralachse 5 wirkende Komponente erhält, wodurch die Seitenwände 19;20 von der Zentralachse 5 weg gegen die Durchgangsbohrung 6 des Verbindungskörpers 2 gepresst werden und somit der Klemmkörper 7 und der Längsträger 1 innerhalb des Verbindungskörpers 2 fixiert werden. Damit das klemmkörperseitige Ende 46 der Feststellschraube 43 auf den Längsträger 1 drückt und nicht etwa die obere Begrenzung 49 des Freiraumes 25, muss die Feststellschraube 43 um eine Länge X in den Verbindungskörper 2 eindringen, das grösser als das Mass Y ist, welches sich aus der Differenz der Höhe H des Verbindungskörpers 2 und der Tiefe T des Freiraumes 25 ergibt. Als Bedingung für diesen Fall der Klemmwirkung gilt:

$$X > H - T$$

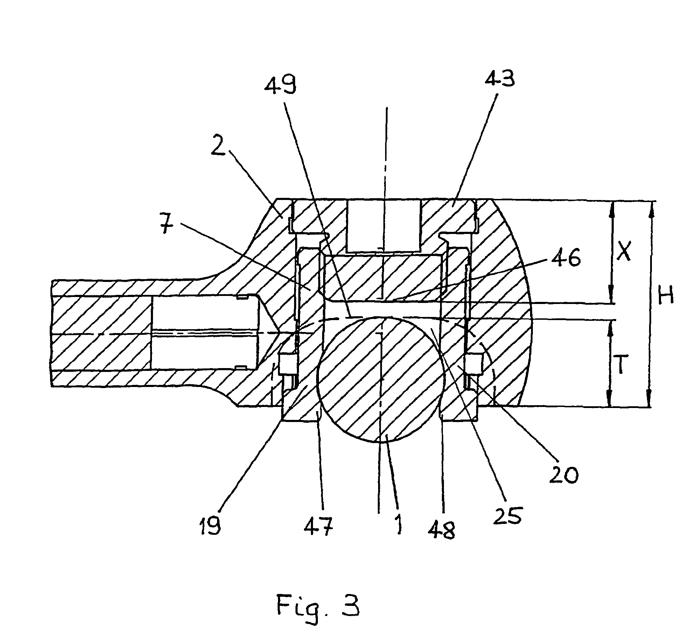

In Fig. 3 ist eine Ausführungsform der erfindungsgemässen Vorrichtung dargestellt, die sich von der in den Fig. 1 und 2 beschriebenen Variante nur dadurch unterscheidet, dass nur die obere Begrenzung 49 des Freiraumes 25 von oben auf den Längsträger 1 drückt und diesen gegen die Erhebungen 47;48 an den Seitenwänden 19;20 des Klemmkörpers 7 presst. Damit die obere Begrenzung 49 des Freiraumes 25 und nicht das klemmkörperseitige Ende 46 der Feststellschraube 43 auf den Längsträger 1 drückt, sind der Verbindungskörper 2 und die Festsstellschraube 43 so beschaffen, dass die Feststellschraube 43 nur um eine Länge X in den Verbindungskörper 2 eindringt, das kleiner als das Mass Y ist, welches sich aus der Differenz der Höhe H des Verbindungskörpers 2 und der Tiefe T des Freiraumes 25 ergibt. Als Bedingung für diesen Fall der Klemmwirkung gilt:

$$X < H - T$$

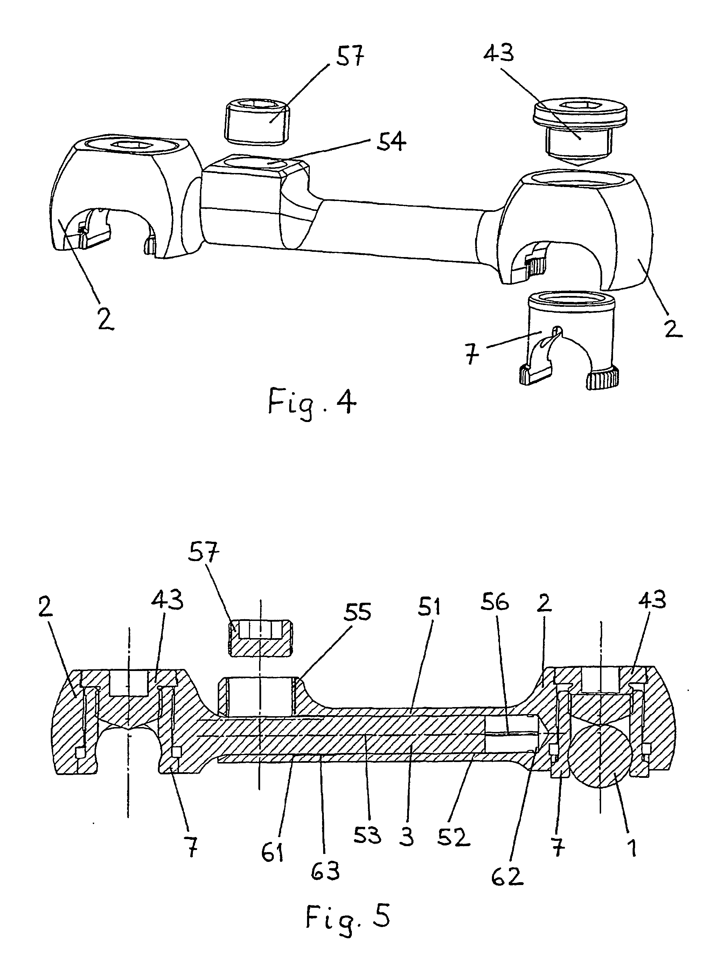

In den Fig. 4 und 5 ist die erfindungsgemässe Vorrichtung in einer Ausführungsform als Querverbinder zwischen zwei Längsträgern 1 dargestellt. Die Vorrichtung besteht in dieser Ausführung aus zwei Verbindungskörpem 2 mit je einem Klemmkörper 7 und je einer Feststellschraube 43. Die beiden Verbindungskörper 2 sind durch Querstäbe 3;51, welche ineinander teleskopierbar sind, miteinander verbunden. Der äussere der Querstäbe 51 ist mittels einer Bohrung 52 mit einer Längsachse 53 hohlzylinderartig gestaltet. Die Bohrung 52 ist so dimensioniert, dass der innere Querstab 3 darin gleitbar und in Richtung der Längsachse 53 verschiebbar ist. Zudem ist der Querstab 51 in der Weise mit einer quer zur Längsachse 53 verlaufenden Bohrung 54 mit Innengewinde 55 versehen, dass mittels einer in das Innengewinde 55 einschraubbaren Fixierschraube 57 die beiden Querstäbe in Richtung der Längsachse 53 relativ zueinander blockierbar sind. Am vom Verbindungskörper 2 entfernten Ende des inneren Querstabes 3 sind über dessen Querschnitt verteilt vier Schlitze 56 so angebracht, dass der innere Querstab 3 in diesem Bereich quer zur Längsachse 53 federbar ist. Ebenfalls am vom Verbindungskörper 2 entfernten Ende des inneren Querstabes 3 angebracht sind ringsektorförmige Anschläge 62, welche einen grösseren Durchmesser als der übrige Querstab 3 aufweisen. Dadurch wird erreicht, dass beim Auseinanderziehen der beiden Querstäbe 3; 51 die quer zur Längsachse 53 federbaren Anschläge 62 in eine Hinterdrehung 63 in der Bohrung 52 einschnappen und bei weiterem Auseinanderziehen der Querstäbe 3;51 an der vom Verbindungskörper 2 entfernten Stirnfläche 61 der Hinterdrehung 63 anschlagen, wodurch ein ungewolltes Auseinandergleiten der beiden Querstäbe 3;51 verhindert wird.

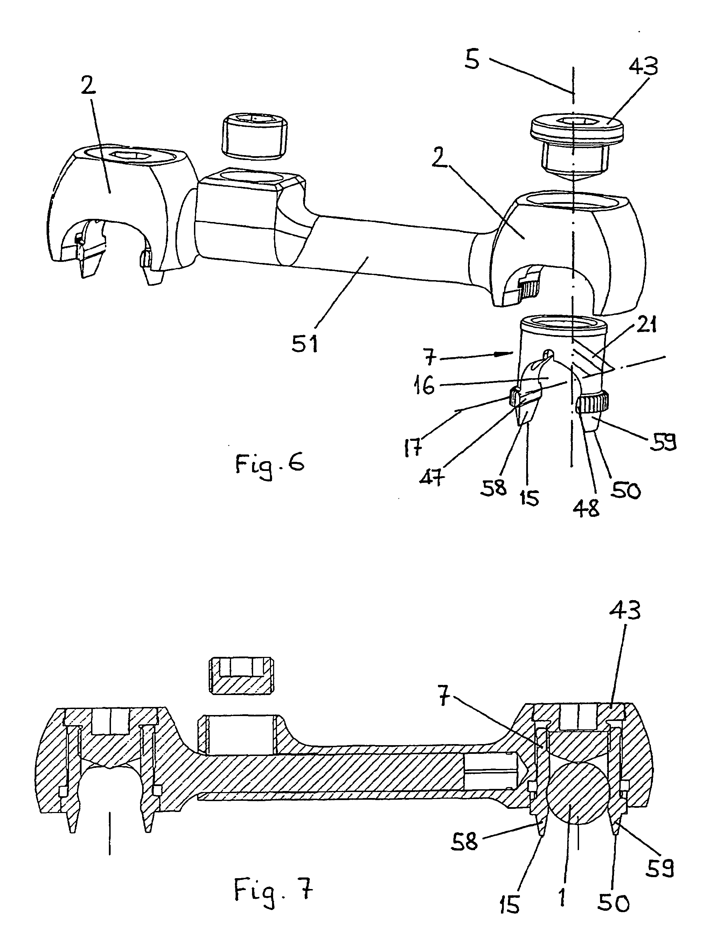

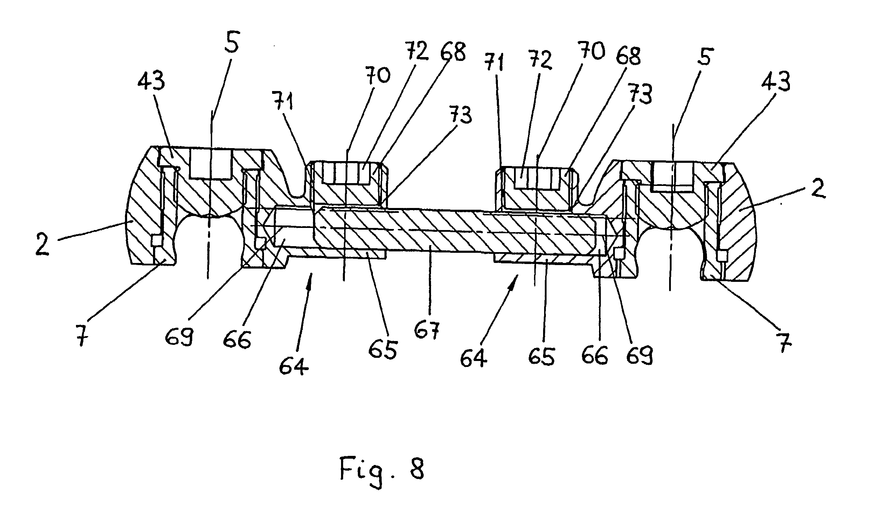

Die in den Fig. 6 und 7 dargestellte Ausführungsform unterscheidet sich von der in den Fig. 4 und 5 dargestellten Ausführungsform nur dadurch, dass der Klemmkörper 7 mit Zentrierbacken 58;59 versehen ist, welche sich an den Abschnitt 40 anschliessen und gegen die freien Enden 15;50 hin erstrecken. Diese Zentrierbacken 58;59 sind so gestaltet, dass sich der Querschnitt der Durchgangsöffnung 16 von den freien Enden 15;50 gegen die Erhebungen 47;48 hin gegen die Zentralachse 5 verengt. Zudem verjüngen sich die Zentrierbacken 58;59 in einem parallel zur Ebene 21 verlaufenden Längsschnitt gegen die freien Enden 15;50 hin. Dadurch wird erreicht, dass der Klemmkörper 7 beim Einführen eines Längsträgers 1 in die Durchgangsöffnung 16 so um die Zentralachse 5 gedreht wird, dass die Längsachse 17 der Durchgangsöffnung 16 parallel zur Achse dieses Längsträgers 1 ausgerichtet wird.

In Fig. 8 ist eine Ausführungsform dargestellt, welche zwei durch einen Stab 67 verbundene Verbindungskörper 2, mit Festetfschraube 43 und Klemmkörper 7 umfasst. Die beiden Verbindungskörper 2 unterscheiden sich von dem in Fig. 1 dargestellten Verbindungskörper 2 nur durch die Ausführung der Kupplungsmittel 64. Jeder Verbindungskörper 2 umfasst in der hier dargestellten Ausführungsform als Kupplungsmittel 64 eine Muffe 65, welche senkrecht zur Zentralachse 5 eine als Sackloch ausgeführte Bohrung 66 mit einer Längsachse 69 aufweist. Ein Stab 67 ist so in die Bohrung 66 einführbar, dass mittels dieses Stabes 67 zwei Verbindungskörper 2 zur Achse 69 fluchtend miteinander verbindbar sind. Zur Befestigung des Stabes 67 in der Bohrung 66 ist die Muffe 65 mit einer Bohrung 73 mit Innengewinde 71 versehen, deren Längsachse 70 quer zur Längsachse 69 verläuft und diese schneidet. In dieses Innengewinde 71 wird eine Stiftschraube 68 eingeschraubt, welche gestattet, den Stab 67 innerhalb der Bohrung 66 zu fixieren. Zum Anziehen und Lösen ist die Stiftschraube 68 mit einem Innensechskant 72 versehen. Da der Stab 67 in der Bohrung 66 verschiebbar ist, ist der Abstand zwischen den beiden am Stab 67 befestigbaren Verbindungskörper 2 innerhalb gewisser Grenzen einstellbar. Die Vorrichtung gemäss Fig. 8 kann mit Klemmkörpern 7 gemäss Fig. 1 oder gemäss den Fig. 6 und 7 ausgestattet sein.


**Patentansprüche**

1. Vorrichtung zum lösbaren Festklemmen und Verbinden eines Längsträgers (1) innerhalb eines chirurgischen Implantates im menschlichen oder tierischen Körper; welche

   A) mindestens einen jochartigen Verbindungskörper (2) mit den Seitenwänden (8) und dem Steg (60), einer den Steg (60) schneidenden, zwischen den Seitenwänden verlaufenden Zentralachse (5), einem zwischen Steg (60) und Seitenwänden (8) liegenden Freiraum (25) für die Durchführung eines Längsträgers (1) quer zur Zentralachse (5) und einer koaxial zur Zentralachse (5) verlaufenden, den Verbindungskörper (2) durchdringenden Durchgangsbohrung (6);
   B) aussen am jochartigen Verbindungskörper (2) quer zur Zentralachse (5) an mindestens einer der Seitenwände (8) angeordnete Kupplungsmittel (64) zur Befestigung des Verbindungskörpers (2) innerhalb des Implantates;
   C) einen in der Durchgangsbohrung (6) gleitbaren, jochartigen Klemmkörper (7) mit quer zur Zentralachse (5) federbaren Seitenwänden (19;20) mit freien Enden (15;50) und einer zwischen diesen Seitenwänden (19;20) angeordneten, den Klemmkörper (7) durchdringenden Durchgangsöffnung (16) mit einer quer zur Zentralachse (5) verlaufenden Längsachse (17), die zur Aufnahme eines Längsträgers (1) dient; und

   D) Fixationsmittel (9) umfasst, welche koaxial zur Zentralachse (5), dem Freiraum (25) gegenüberliegend so am Verbindungskörper (2) anlegbar sind und mit dem Klemmkörper (7) schraubbar verbindbar sind, dass durch Anziehen der Fixationsmittel (9) eine koaxial zur Zentralachse (5) gerichtete Zugkraft auf den Klemmkörper (7) ausübbar ist, wobei
   E) die Seitenwände (19;20) des Klemmkörpers (7) gegen ihre freien Enden (15;50) hin mit gegen die Zentralachse (5) gerichteten Erhebungen (47;48) versehen sind, so dass die Durchgangsöffnung (16) im Querschnitt betrachtet eine Begrenzung mit einem Zentriwinkel von mehr als 180° aufweist; und
   F) der Klemmkörper (7) relativ zum Verbindungskörper (2) in eine erste Position bringbar ist, welche ein Ausfedern der Seitenwände (19;20) quer zur Zentralachse (5) gestattet, so dass ein Längsträger (1) koaxial zur Längsachse (17) in die Durchgangsöffnung (16) einschnappbar ist,
   **dadurch gekennzeichnet, dass**
   G) die Seitenwände (19;20) parallel zur Zentralachse (5) verlaufen; und
   H) der Klemmkörper (7) durch Anziehen der Fixationsmittel (9) in eine zweite Position bringbar ist, so dass der Querschnitt der Durchgangsöffnung (16) von oben parallel zur Zentralachse (5) durch mindestens eines der relativ zum Klemmkörper (7) bewegten Teile (2;43) der Vorrichtung so weit verkleinert wird, dass ein in der Durchgangsöffnung (16) eingelegter Längsträger (1) gegen die Erhebungen (47;48) gepresst wird, wodurch eine Keilwirkung auf die Erhebungen (47;48) entsteht und die Seitenwände (19;20) des Klemmkörpers (7) gegen die Innenseiten der Seitenwände (8) des Verbindungskörpers (2) gepresst werden.

2. Vorrichtung nach Anspruch 1, **dadurch gekennzeichnet, dass** die Durchgangsöffnung (16) im Querschnitt einen Zentriwinkel von zwischen 200° und 250° aufweist.

3. Vorrichtung nach Anspruch 1 oder 2, **dadurch gekennzeichnet, dass** die Durchgangsöffnung (16) im Querschnitt einen Zentriwinkel von zwischen 210° und 240° aufweist.

4. Vorrichtung nach einem der Ansprüche 1 bis 3, **da-**

**durch gekennzeichnet, dass** der Klemmkörper (7) und die Durchgangsbohrung (6) am Umfang mit korrespondierenden Verzahnungen (37;38) versehen sind, wobei die Zähne dieser Verzahnungen (37;38) parallel zur Zentralachse (5) verlaufen.

5. Vorrichtung nach einem der Ansprüche 1 bis 4, **dadurch gekennzeichnet, dass** der Verbindungskörper (2) in Richtung der Zentralachse (5) ein klemmkörperseitiges Ende (28) und ein entgegengesetztes fixationsmittelseitiges Ende (27) aufweist, wobei der zwischen den Seitenwänden (8) liegende Freiraum (25) gegen das klemmkörperseitige Ende (28) offen ist, und durch das Fixationsmittel (9) vom fixationsmittelseitigen Ende (27) des Verbindungskörpers (2) her eine Zugkraft auf den Klemmkörper (7) ausübbar ist.

6. Vorrichtung nach Anspruch 5, **dadurch gekennzeichnet, dass** der Klemmkörper (7) an seinem fixationsmittelseitigen Ende (14) mit einem koaxial zur Zentralachse (5) verlaufenden Innengewinde (12) versehen ist und das Fixationsmittel (9) eine Feststellschraube (43) ist, welche ein zum Innengewinde (12) korrespondierendes Aussengewinde (13) aufweist und deren Schraubenkopf (41) am fixationsmittelseitigen Ende (27) des Verbindungskörper (2) gegen eine Bewegung parallel zur Zentralachse (5) gesichert ist.

7. Vorrichtung nach Anspruch 6, **dadurch gekennzeichnet, dass** die Feststellschraube (43) in Richtung der Zentralachse (5) um eine Länge X vom fixationsmittelseitigen Ende (27) her in den Verbindungskörper (2) eindringt, der Verbindungskörper (2) parallel zur Zentralachse (5) zwischen seinem fixationsmittelseitigen Ende (27) und seinem klemmkörperseitigen Ende (28) eine Höhe H aufweist, der Freiraum (25) quer zur Zentralachse (5) gegen den Steg (60) eine Begrenzung (49) aufweist, so dass der Freiraum (25) zwischen dem klemmkörperseitigen Ende (28) und der Begrenzung (49) eine Tiefe T aufweist und die Bedingung

$$X > H - T$$

erfüllt ist.

8. Vorrichtung nach Anspruch 6, **dadurch gekennzeichnet, dass** die Feststellschraube (43) in Richtung der Zentralachse (5) um eine Länge X vom fixationsmittelseitigen Ende (27) her in den Verbindungskörper (2) eindringt, der Verbindungskörper (2) in Richtung der Zentralachse (5) zwischen seinem fixationsmittelseitigen Ende (27) und seinem klemmkörperseitigen Ende (28) eine Höhe H aufweist, der Freiraum (25) quer zur Zentralachse (5) gegen den Steg (60) eine Begrenzung (49) aufweist, so dass der Freiraum (25) zwischen dem klemmkörperseitigen Ende (28) und der Begrenzung (49) eine Tiefe T aufweist und die Bedingung

$$X < H - T$$

erfüllt ist.

9. Vorrichtung nach einem der Ansprüche 1 bis 8, **dadurch gekennzeichnet, dass** die Durchgangsöffnung (16) einen Kem mit einem kreissegmentartigen Querschnitt aufweist, wobei der Zentriwinkel des Kreissegmentes mehr als 180° beträgt.

10. Vorrichtung nach Anspruch 9, **dadurch gekennzeichnet, dass** der Kem der Durchgangsöffnung (16) einen Durchmesser D aufweist und die Erhebungen (47;48) gegen die Durchgangsöffnung (16) hin durch Seitenflächen (22;23) begrenzt sind, welche rechtwinklig zur Zentralachse (5) einen Abstand A aufweisen, wobei das Verhältnis A/D zwischen 85 % und 98 % beträgt.

11. Vorrichtung nach Anspruch 10, **dadurch gekennzeichnet, dass** das Verhältnis A/D zwischen 92 % und 98 % beträgt.

12. Vorrichtung nach einem der Ansprüche 1 bis 11, **dadurch gekennzeichnet, dass** die Erhebungen (47; 48) keilförmig gestaltet sind, wobei sich jede Erhebung (47;48) parallel zur Zentralachse (5) und gegen den Kem der Durchgangsöffnung (16) verjüngt.

13. Vorrichtung nach einem der Ansprüche 1 bis 12, **dadurch gekennzeichnet, dass** die Kupplungsmittel (64) eine Muffe (65) mit einer quer zur Zentralachse (5) angeordneten Bohrung (66) zur Aufnahme eines Stabes (67) und die Muffe (65) durchdringend eine Befestigungsschraube (68) zur Fixierung des Stabes (67) in der Bohrung (66) umfassen.

14. Vorrichtung nach einem der Ansprüche 1 bis 12, **dadurch gekennzeichnet, dass** die Kupplungsmittel (64) einen quer zur Zentralachse (5) am Verbindungskörper (2) angeordneten Querstab (3;51) umfassen.

15. Vorrichtung nach einem der Ansprüche 1 bis 14, **dadurch gekennzeichnet, dass** die freien Enden (15; 50) des Klemmkörpers (7) mit Zentrierbacken (58; 59), welche eine einfache Zentrierung und Einführung eines Längsträgers (1) in die Durchgangsöffnung (16) ermöglichen, versehen sind.

16. Vorrichtung nach Anspruch 15, **dadurch gekennzeichnet, dass** die Zentrierbacken (58;59) so gestaltet sind, dass sich der Querschnitt der Durchgangsöffnung (16) von den freien Enden (15;50) zu den Erhebungen (47;48) hin verjüngt.

17. Vorrichtung nach Anspruch 15 oder 16, **dadurch gekennzeichnet, dass** sich die Zentrierbacken (58;59) in parallel zu einer Ebene (21), welche durch die Zentralachse (5) und die Längsachse (17) aufgespannt wird, verlaufenden Ebenen gegen die freien Enden (15;50) hin verjüngen.

18. Vorrichtung nach einem der Ansprüche 1 bis 17, **dadurch gekennzeichnet, dass** sie zusätzlich einen zweiten Verbindungskörper (2) mit Klemmkörper (7) und Feststellschraube (43) umfasst.

19. Vorrichtung nach Anspruch 18, **dadurch gekennzeichnet, dass** die beiden Verbindungskörper (2) durch Querstäbe (3;51), welche ineinander teleskopierbar sind, miteinander verbunden sind.

20. Vorrichtung nach Anspruch 18, **dadurch gekennzeichnet, dass** die beiden Verbindungskörper (2) durch einen Stab (67), welcher in die Bohrungen (66) der Muffen (65) einführbar ist und mittels der Befestigunsschrauben (68) fixierbar ist, verbunden sind.

21. Vorrichtung nach einem der Ansprüche 1 bis 17, **dadurch gekennzeichnet, dass** sie mittels der Kupplungsmittel (64) mit einem Knochenverankerungselement verbindbar ist.

22. Vorrichtung nach einem der Ansprüche 1 bis 17, **dadurch gekennzeichnet, dass** sie mittels der Kupplungsmittel (64) mit einer Pediketschraube verbindbar ist.

23. Vorrichtung nach einem der Ansprüche 1 bis 22, **dadurch gekennzeichnet, dass** sie zusätzlich einen Längsträger (1) umfasst, wobei

A) beim Anziehen des Fixationsmittels (9) der Klemmkörper (7) so weit gegenüber dem Verbindungskörper (2) und dem Fixationsmittel (9) bewegt wird, bis der in der Durchgangsöffnung (16) eingebrachte Längsträger (1) an einem dieser relativ zum Klemmkörper (7) bewegten Teile (2;43) der Vorrichtung zur Anlage kommt, wodurch der Längsträger (1) gegen die Erhebungen (47;48) gedrückt wird; und
B) durch die senkrecht zur Zentralachse (5) gerichtete Komponente der durch den Längsträger (1) auf die Erhebungen (47;48) ausgeübten Druckkraft die Seitenwände (19;20) des Klemmkörpers (7) gegen die Wand der Durchgangsbohrung (6) gepresst werden.

**Claims**

1. Device for releasably clamping and connecting a longitudinal member (1) within a surgical implant in the body of human or animal, which comprises

A) at least one yoke-like connecting body (2) with the side walls (8) and the cross-piece (60), a central axis (5) intersecting the cross-piece (60) and running between the side walls, a free space (35) lying between the cross-piece (60) and side walls (8) for leading through a longitudinal member (1) transversely with respect to the central axis (5), and a through-bore (6) running coaxially with respect to the central axis (5) and penetrating the connecting body (2);
B) coupling means (64) arranged on the outside of the yoke-like connecting body (2) transversely with respect to the central axis (5) on at least one of the side walls (8) for fastening the connecting body (2) within the implant;
C) a yoke-like clamping body (7) which can slide in the through-bore (6) and has side walls (19; 20) resilient transversely with respect to the central axis (5), with free ends (15; 50), and a through-opening (16) arranged between these side walls (19; 20) and penetrating the clamping body (7), with a longitudinal axis (17) running transversely with respect to the central axis (5), which serves for receiving a longitudinal member (1); and
D) fixation means (9) which can be placed on the connecting body (2) coaxially with respect to the central axis (5) and opposite the free space (25) and can be connected to the clamping body (7) in a screwable manner in such a way that, by tightening the fixation means (9), a tensile force directed coaxially with respect to the central axis (5) can be exerted on the clamping body (7),whereby
E) the side walls (19; 20) of the clamping body (7) are provided towards their free ends (15; 50) with elevations (47; 48) directed towards the central axis (5), so that the through-opening (16) has a periphery with a central angle of more than 180° when viewed in cross section; and
F) the clamping body (7) can be brought into a first position in relation to the connecting body (2) which allows the side walls (19; 20) to spring out transversely with respect to the central axis (5), so that a longitudinal member (1) can be snapped into the through-opening (16) coaxially with respect to the longitudinal axis (17);
**characterised in that**
G) the side walls (19;20) run parallel to the central axis (5); and
H) by tightening the fixation means (9), the clamping body (7) can be brought into a second position, so that the cross section of the through-opening is reduced from above parallel to the central axis (5) by at least one of the parts (2; 43) moved in relation to the clamping body (7) to such an extent that a longitudinal member (I) placed in the through-opening (16) is pressed against the elevations (47; 48), whereby a wedging effect on the elevations (47; 48) is produced and the side walls (19; 20) of the clamping body (7) are pressed against the inner sides of the side walls (8) of the connecting body (2).

2. Device according to Claim 1, **characterized in that** the through-opening (16) has in cross section a central angle of between 200° and 250°.

3. Device according to Claim 1 or 2, **characterized in that** the through-opening (16) has in cross section a central angle of between 210° and 240°.

4. Device according to one of Claims 1 to 3, **characterized in that** the clamping body (7) and the through-bore (6) are provided on the circumference with corresponding serrations (37; 38), the teeth of these serrations (37; 38) running parallel to the central axis (5).

5. Device according to one of Claims 1 to 4, **characterized in that** the connecting body (2) has in the direction of the central axis (5) an end (28) on the clamping body side and an opposite end (27) on the fixation means side, the free space (25) lying between the side walls (8) being open towards the end (28) on the clamping body side, and it being possible to exert a tensile force on the clamping body (7) by the fixation means (9) from the end (37) of the connecting body (2) on the fixation means side.

6. Device according to Claim 5, **characterized in that** the clamping body (7) is provided at its end (14) on the fixation means side with an internal thread (12) running coaxially with respect to the central axis (5) and the fixation means (9) is a locking screw (43) which has an external thread (13) corresponding to the internal thread (12) and the screw head (41) of which at the end (27) of the connecting body (2) on the fixation means side is secured against a movement parallel to the central axis (5).

7. Device according to Claim 6, **characterized in that** the locking screw (43) penetrates into the connecting body (2) in the direction of the central axis (5) by a length X from the end (27) on the fixation means side, the connecting body (2) has a height H parallel to the central axis (5) between its end (27) on the fixation means side and its end (28) on the clamping body side, the free space (25) has a limitation (49) transversely with respect to the central axis (5) towards the cross-piece (60), so that the free space (25) has a depth T between the end (28) on the clamping body side and the limitation (49), and the condition

$$X > H - T$$

is satisfied.

8. Device according to Claim 6, **characterized in that** the locking screw (43) penetrates into the connecting body (2) in the direction of the central axis (5) by a length X from the end (27) on the fixation means side, the connecting body (2) has a height H in the direction of the central axis (5) between its end (27) on the fixation means side and its end (28) on the clamping body side, the free space (25) has a limitation (49) transversely with respect to the central axis (5) towards the cross-piece (60), so that the free space (25) has a depth T between the end (28) on the clamping body side and the limitation (49), and the condition

$$X < H - T$$

is satisfied.

9. Device according to one of Claims 1 to 8, **characterized in that** the through-opening (16) has a core with a cross section similar to a segment of a circle, the central angle of the segment of a circle being more than 180°.

10. Device according to Claim 9, **characterized in that** the core of the through-opening (16) has a diameter D and the elevations (47; 48) are bounded towards the through-opening (16) by side faces (22; 23) which have a distance A at right angles with respect to the central axis (5), the ratio A/D being between 85% and 98%.

11. Device according to Claim 10, **characterized in that** the ratio A/D is between 92% and 98%.

12. Device according to one of Claims 1 to 11, **characterized in that** the elevations (47; 48) are shaped in the form of wedges, each elevation (47; 48) tapering parallel to the central axis (5) and towards the core of the through-opening (16).

13. Device according to one of Claims 1 to 12, **characterized in that** the coupling means (64) comprise a sleeve (65) with a bore (66) arranged transversely with respect to the central axis (5) for receiving a rod (67) and, penetrating the sleeve (65), a fastening screw (68) for fixing the rod (67) in the bore (66).

14. Device according to one of Claims 1 to 12, **characterized in that** the coupling means (64) comprise a transverse rod (3; 51) arranged transversely with respect to the central axis (5) on the connecting body (2).

15. Device according to one of Claims 1 to 14, **characterized in that** the free ends (15; 50) of the clamping body (7) are provided with centring jaws (58; 59) which make it possible for a longitudinal member (1) to be centred and inserted into the through-opening (16) in a simple manner.

16. Device according to Claim 15, **characterized in that** the centring jaws (58; 59) are shaped in such a way that the cross section of the through-opening (16) tapers from the free ends (15; 50) towards the elevations (47; 48).

17. Device according to Claim 15 or 16, **characterized in that** the centring jaws (58; 59) taper towards the free ends (15; 50) in planes running parallel to a plane (21) which is defined by the central axis (5) and the longitudinal axis (17).

18. Device according to one of Claims 1 to 17, **characterized in that** it additionally comprises a second connecting body (2) with a clamping body (7) and locking screw (43).

19. Device according to Claim 18, **characterized in that** the two connecting bodies (2) are connected to one another by transverse rods (3; 51) which can be telescoped in one another.

20. Device according to Claim 18, **characterized in that** the two connecting bodies (2) are connected by a rod (67) which can be inserted into the bores (66) of the sleeves (65) and can be fixed by means of the fastening screws (68).

21. Device according to one of Claims 1 to 17, **characterized in that** it is connected by means of the coupling means (64) to a bone anchorage element.

22. Device according to one of Claims 1 to 17, **characterized in that** it is connected by means of the coupling means (64) to a pedicle screw.

23. Device according to one of Claims 1 to 22, **characterized in that** it additionally comprises a longitudinal member (1), wherein,

A) when the fixation means (9) is tightened, the clamping body (7) is moved with respect to the connecting body (2) and the fixation means (9) until the longitudinal member (1) inserted in the through-opening (16) comes to bear against one of these parts (2; 43) of the device moved in relation to the clamping body (7), whereby the longitudinal member (1) is pressed against the elevations (47; 48); and
B) the side walls (19; 20) of the clamping body (7) are pressed against the wall of the through-bore (6) by the component directed perpendicularly with respect to the central axis (5) of the compressive force exerted by the longitudinal member (1) on the elevations (47; 48).

**Revendications**

1. Dispositif pour le blocage et la liaison amovibles d'un support longitudinal (1) au sein d'un implant chirurgical dans le corps humain ou animal; comprenant

A) au moins un organe de liaison (2) en forme d'arceau avec les parois latérales (8) et l'arc (60), un axe central (5) traversant l'arc (60) entre les parois latérales, un espace (25) situé entre les parois latérales (8) et l'arc (60) et destiné à faire passer un support longitudinal (1) transversalement à l'axe central (5), et un alésage traversant (6) coaxial par rapport à l'axe central (5) et qui traverse l'organe de liaison (2);
B) sur l'extérieur de l'organe de liaison (2) en forme d'arceau, des éléments d'accouplement (64) placés sur au moins une des parois latérales (8) transversalement à l'axe central (5) et destinés à fixer l'organe de liaison (2) au sein de l'implant;
C) un organe de serrage (7) en forme d'arceau, mobile dans l'alésage traversant (6), avec des parois latérales (19;20) élastiques transversalement à l'axe central (5), qui ont des extrémités libres (15;50) et un trou traversant (16) placé entre ces parois latérales (19;20), qui traverse l'organe de serrage (7), a un axe longitudinal (17) perpendiculaire à l'axe central (5) et sert à recevoir un support longitudinal (1); et
D) des éléments de fixation (9), pouvant adhérer à l'organe de liaison (2) coaxialement à l'axe central (5) et en face de l'espace (25) et pouvant être vissés à l'organe de serrage (7), de telle sorte qu'on peut, en serrant les éléments de fixation (9), exercer sur l'organe de serrage (7) une force de traction coaxiale par rapport à l'axe central (5),
E) les parois latérales (19;20) de l'organe de serrage (7) étant munies vers leurs extrémités libres (15;50) de saillies (47;48) orientées vers l'axe central (5), de sorte que la section transversale du trou traversant (16) présente une limite avec un angle au centre supérieur à 180°; et

F) l'organe de serrage (7) pouvant être amené dans une première position par rapport à l'organe de liaison (2), cette position permettant une détente des parois latérales (19;20) transversalement à l'axe central (5), de sorte qu'un support longitudinal (1) peut s'encliqueter dans le trou traversant (16) coaxialement à l'axe longitudinal (17),

**caractérisé en ce que**

G) les parois latérales (19;20) sont parallèles à l'axe central (5); et **en ce qu'**on peut

H) amener l'organe de serrage (7) dans une deuxième position en serrant les éléments de fixation (9), de façon à réduire la section transversale du trou traversant (16) du haut parallèlement à l'axe central (5), grâce à au moins une des pièces (2;43) du dispositif mobiles par rapport à l'organe de serrage (7), et ce de telle sorte qu'un support longitudinal (1) introduit dans le trou traversant (16) soit pressé contre les saillies (47;48), ce qui entraîne un effet de coin sur les saillies (47;48) et presse les parois latérales (19;20) de l'organe de serrage (7) contre les faces internes des parois latérales (8) de l'organe de liaison (2).

2. Dispositif selon la revendication 1, **caractérisé en ce que** la section transversale du trou traversant (16) présente un angle au centre compris entre 200° et 250°.

3. Dispositif selon la revendication 1 ou 2, **caractérisé en ce que** la section transversale du trou traversant (16) présente un angle au centre compris entre 210° et 240°.

4. Dispositif selon une des revendications 1 à 3, **caractérisé en ce que** l'organe de serrage (7) et l'alésage traversant (6) portent sur leur pourtour des dentures correspondantes (37;38), les dents de ces dentures (37;38) étant parallèles à l'axe central (5).

5. Dispositif selon une des revendications 1 à 4, **caractérisé en ce que** l'organe de liaison (2) présente, selon la direction de l'axe central (5), une extrémité (28) située du côté de l'organe de serrage et une extrémité (27) opposée située du côté de l'élément de fixation (27), l'espace (25) situé entre les parois latérales (8) étant ouvert vers l'extrémité (28) située du côté de l'organe de serrage, et **en ce que** l'élément de fixation (9) peut exercer une force de traction sur l'organe de serrage (7) depuis l'extrémité (27) de l'organe de liaison (2) située du côté de l'élément de fixation.

6. Dispositif selon la revendication 5, **caractérisé en ce que** l'extrémité (14) de l'organe de serrage (7) située du côté de l'élément de fixation est munie d'un filetage femelle (12) coaxial par rapport à l'axe central (5) et **en ce que** l'élément de fixation (9) est une vis de blocage (43) qui présente un filetage mâle (13) correspondant au filetage femelle (12) et dont la tête de vis (41) est, sur l'extrémité (27) de l'organe de liaison (2) située du côté de l'élément de fixation, bloquée contre un mouvement parallèle à l'axe central (5).

7. Dispositif selon la revendication 6, **caractérisé en ce que** la vis de blocage (43) pénètre dans l'organe de liaison (2) sur une longueur X selon la direction de l'axe central (5), depuis l'extrémité (27) située du côté de l'élément de fixation, **en ce que** l'organe de liaison (2) présente, parallèlement à l'axe central (5), une hauteur H entre son extrémité (27) située du côté de l'élément de fixation et son extrémité (28) située du côté de l'organe de serrage, **en ce que** l'espace (25) présente vers l'arc (60) une limite (49) perpendiculaire à l'axe central (5), de telle sorte que l'espace (25) présente une profondeur T entre l'extrémité (28) située du côté de l'organe de serrage et la limite (49) et que la condition

$$X > H - T$$

est remplie.

8. Dispositif selon la revendication 6, **caractérisé en ce que** la vis de blocage (43) pénètre dans l'organe de liaison (2) sur une longueur X selon la direction de l'axe central (5), depuis l'extrémité (27) située du côté de l'élément de fixation, **en ce que** l'organe de liaison (2) présente, selon la direction de l'axe central (5), une hauteur H entre son extrémité (27) située du côté de l'élément de fixation et son extrémité (28) située du côté de l'organe de serrage, **en ce que** l'espace (25) présente vers l'arc (60) une limite (49) perpendiculaire à l'axe central (5), de telle sorte que l'espace (25) présente une profondeur T entre l'extrémité (28) située du côté de l'organe de serrage et la limitation (49) et que la condition

$$X < H - T$$

est remplie.

9. Dispositif selon une des revendications 1 à 8, **caractérisé en ce que** le trou traversant (16) présente une âme dont la section transversale a la forme d'un segment de cercle, l'angle au centre du segment de cercle étant supérieur à 180°.

**10.** Dispositif selon la revendication 9, **caractérisé en ce que** l'âme du trou traversant (16) présente un diamètre D et que les saillies (47;48) sont limitées vers le trou traversant (16) par des faces latérales (22;23) qui, transversalement à l'axe central (5), présentent un écart A, le rapport A/D étant compris entre 85 % et 98 %.

**11.** Dispositif selon la revendication 10, **caractérisé en ce que** le rapport A/D est compris entre 92 % et 98 %.

**12.** Dispositif selon une des revendications 1 à 11, **caractérisé en ce que** les saillies (47;48) sont cunéiformes, chaque saillie (47;48) rétrécissant parallèlement à l'axe central (5) et vers l'âme du trou traversant (16).

**13.** Dispositif selon une des revendications 1 à 12, **caractérisé en ce que** les éléments d'accouplement (64) comprennent un manchon (65) avec un alésage (66) perpendiculaire à l'axe central (5) et destiné à recevoir une tige (67) et **en ce que** le manchon (65) est traversé par une vis de fixation (68) destinée à fixer la tige (67) dans l'alésage (66).

**14.** Dispositif selon une des revendications 1 à 12, **caractérisé en ce que** les éléments d'accouplements (64) comprennent une tige transversale (3;51) perpendiculaire à l'axe central (5) placée sur l'organe de liaison (2).

**15.** Dispositif selon une des revendications 1 à 14, **caractérisé en ce que** les extrémités libres (15;50) de l'organe de serrage (7) sont munies de mâchoires de centrage (58;59) qui permettent le centrage et l'introduction faciles d'un support longitudinal (1) dans le trou traversant (16).

**16.** Dispositif selon la revendication 15, **caractérisé en ce que** les mâchoires de centrage (58;59) sont conçues de telle sorte que la section transversale du trou traversant (16) rétrécit des extrémités libres (15;50) vers les saillies (47;48)

**17.** Dispositif selon la revendication 15 ou 16, **caractérisé en ce que** les mâchoires de centrage (58;59) rétrécissent vers les extrémités libres (15;50), dans des plans parallèles à un plan (21) formé par l'axe central (5) et l'axe longitudinal (17).

**18.** Dispositif selon une des revendications 1 à 17, **caractérisé en ce qu'**il comprend en plus un deuxième organe de liaison (2) avec organe de serrage (7) et vis de blocage (43)

**19.** Dispositif selon la revendication 18, **caractérisé en ce que** les deux organes de liaison (2) sont reliés par des tiges transversales (3;51) télescopiques l'une dans l'autre.

**20.** Dispositif selon la revendication 18, **caractérisé en ce que** les deux organes de liaison (2) sont reliés par une tige (67) qu'on peut introduire dans les alésages (66) des manchons (65) et fixer à l'aide des vis de fixation (68).

**21.** Dispositif selon une des revendications 1 à 17, **caractérisé en ce qu'**il peut être relié à un élément d'ancrage des os à l'aide des éléments d'accouplement (64).

**22.** Dispositif selon une des revendications 1 à 17, **caractérisé en ce qu'**il peut être relié à une vis pédiculaire à l'aide des éléments d'accouplement (64).

**23.** Dispositif selon une des revendications 1 à 22, **caractérisé en ce qu'**il comprend en plus un support longitudinal (1), tel que

A) lorsqu'on serre l'élément de fixation (9), l'organe de serrage (7) se déplace par rapport à l'organe de liaison (2) et à l'élément de fixation (9) jusqu'à ce que le support longitudinal (1) introduit dans le trou traversant (16) vienne adhérer à une de ces pièces (2;43) du dispositif mobiles par rapport à l'organe de serrage (7), ce qui appuie le support longitudinal (1) contre les saillies (47;48); et
B) la composante de la force de pression exercée par le support longitudinal (1) sur les saillies (47;48) et perpendiculaire à l'axe central (5) presse les parois latérales (19;20) de l'organe de serrage (7) contre la paroi de l'alésage traversant (6).

Fig. 1

Fig.2

Fig. 3

Fig. 4

Fig. 5

Fig. 6

Fig. 7

Fig. 8